# EUROPEAN PATENT APPLICATION

(11) **EP 4 764 494 A1**
(43) Date of publication of application: **24.06.2026**
(21) Application number: 24222613.2
(22) Date of filing: 20.12.2024
(51) Int. Cl.: G01N 33/543, C12Q 1/6816

(54) **CONJUGATES FOR ANALYTE DETECTION AND QUANTIFICATION**

(71) Applicant: Plectonic Biotech GmbH, 85748 Garching bei München (DE)
(72) Inventor: Wagenbauer, Klaus, 85375 Neufahrn (DE); Kick, Benjamin, 85368 Moosburg (DE); Funke, Jonas, 85748 Garching bei München (DE)
(74) Representative: Dentons Patent Solutions Rechtsanwaltsgesellschaft mbH

(57) **Abstract**

The invention relates to a conjugate comprising or consisting of (a) at least one molecule capable of specifically binding to at least one analyte; and at least one nucleic acid nanostructure (b); wherein said nucleic acid nanostructure of (b) comprises a plurality of single-stranded oligonucleotides; and wherein at least two of said plurality of oligonucleotides each comprise a first identical nucleobase sequence.

## Description

### TECHNICAL FIELD OF THE INVENTION

The invention relates to a conjugate comprising or consisting of (a) at least one molecule capable of specifically binding to at least one analyte; and (b) at least one nucleic acid nanostructure; wherein said nucleic acid nanostructure of (b) comprises a plurality of single-stranded oligonucleotides; and wherein at least two of said plurality of oligonucleotides each comprise a first identical nucleobase sequence. The present invention also relates to the use of the conjugate for detecting and/or quantifying at least one analyte. Moreover, the present invention further relates to a method of detecting and/or quantifying at least one analyte. Furthermore, the present invention relates to a kit for the detection and/or quantifying at least one analyte.

### BACKGROUND OF THE INVENTION

In this specification, several documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

DNA nanostructures have garnered a large amount of interest in many fields including clinical diagnosis, healthcare, and environment monitoring. As disclosed in US 7,842,793 B3, new methods for manufacturing nanostructures such as nucleic acid nanostructures have been developed, e.g. "DNA origami". DNA origami uses DNA molecules to produce three-dimensional structures on the nanometer scale. These biodegradable DNA nanostructures are easily programmable into precise geometric configurations and can be functionalized to display a range of biological agents on their surface. Utilizing the programmable nature of DNA base pairing and functionalized strands, many different functionalities can be introduced on the nanoscale.

Structural properties of nucleic acid sequences are inherent characteristics that make nucleic acid nanostructures attractive systems in methods for detecting and/or quantifying a specific analyte. DNA origami technology has seen substantial development in recent years because DNA has numerous merits such as good stability, strong predictability, excellent programmability, easy synthesis, and biocompatibility.

For this reason, functional DNAs have also been designed and applied in the biosensor field, capable of single-molecule detection of DNA and RNA (Zhang et al., 2010a), single nucleotide polymorphisms (Zhang et al., 2010b), as well as various protein biomarkers (Rinker et al., 2008; Koirala et al., 2014) among many others (Wang et al., 2017, Wang et al., 2020).

Nevertheless, there is a great need in the field of molecular diagnostics suitable to detect biomaterials such as proteins, peptides, nucleic acids, macromolecules, and hormones with high contrast and sensitivity. The methodologies available today require expensive equipment and these methods exhibit shortcomings in terms of specificity and sensitivity.

Therefore, a novel approach to detect targets and analytes in a sample with a high dynamic range of a signal and minimized non-specific interaction would be highly desirable to extend these kinds of molecular diagnostics to a large variety of applications and to address novel targets or low abundance biomarkers due to enhanced reagent performance.

In view of the prior art and its shortcomings, the technical problem underlying the present invention can be seen as the provision of improved means and methods for detecting and/or quantifying an analyte in a sample.

This technical problem has been solved by the subject-matter of the claims, as demonstrated by the Examples.

### BRIEF DESCRIPTION OF THE INVENTION

Generally, and by way of brief description, the main aspects of the present invention can be described as follows:
In a first aspect, the present invention provides a conjugate comprising or consisting of
(a) at least one molecule capable of specifically binding to at least one analyte; and
(b) at least one nucleic acid nanostructure;
wherein said nucleic acid nanostructure of (b) comprises a plurality of single-stranded oligonucleotides, and wherein at least two of said plurality of oligonucleotides each comprise a first identical nucleobase sequence.

In a second aspect, the present invention provides a use of a conjugate of the first aspect for detecting and/or quantifying an analyte.

In a third aspect, the present invention provides a method of detecting and/or quantifying an analyte, said method comprising:
(a) allowing formation of a complex of a conjugate as defined above in the first aspect and said analyte; and
(b) in said complex, detecting and/or quantifying the amount of said first complementary strands or said second complementary strand, respectively.

In a fourth aspect, the present invention provides a kit comprising or consisting of the conjugate of the first aspect for detecting and/or quantifying an analyte and primers as defined in the third aspect.

### DETAILED DESCRIPTION

The development of programmable biomaterials such as nucleic acid nanostructures for use *in vitro* and *in vivo* assays represents a major advance for the future of diagnostics. Nucleic acid nanostructures have great potential in biomedical applications, particularly because they are biodegradable, can be engineered to undergo allosteric conformational changes, and allow for precise interactions with target molecules and cells. Conjugates comprising nucleic acid nanostructures which reduces costs and time in diagnostic applications would be highly advantageous in molecular diagnostics.

Therefore, the inventors have developed conjugates comprising modular nucleic acid nanostructures to provide user-defined biomarker detection in one molecule with high specificity and sensitivity. The nanostructure methodology allows the creation of user-defined biocompatible structures providing universal solutions to a variety of biomarkers with seamless integration into existing workflows, instruments, or protocols.

The conjugates of the invention serve as readily available, cost-effective, and modular alternatives to protein scaffolds, especially in settings where high specificity and sensitivity in sample detection is required, e.g. to address novel targets or low abundance analytes or biomarkers.

### Conjugates for analyte detection and quantification

In a first aspect, the invention relates to a conjugate comprising or consisting of
(a) at least one molecule capable of specifically binding to at least one analyte; and (b) at least one nucleic acid nanostructure; wherein
said nucleic acid nanostructure of (b) comprises a plurality of single-stranded oligonucleotides, and at least two of said plurality of oligonucleotides each comprise a first identical nucleobase sequence.

This invention generally relates to conjugates comprising nanostructures. The term "nanostructure" or "nucleic acid nanostructure", as used herein, relates to nucleic acids that form (e.g., self-assemble) two-dimensional (2D) or three-dimensional (3D) shapes.

The term "nucleic acid", as used herein, has its art-established meaning, and relates to polycondensates of nucleotides, such as RNA or DNA. In one example, the nucleic acid nanostructures may be made of, or comprise, DNA, RNA, modified DNA, modified RNA, PNA, LNA or a combination thereof.

In one example, the at least one nucleic acid nanostructures of the invention are comprised of oligonucleotides that are bound to each other in a sequence-specific manner to form 3D shapes (Zadegan RM and Norton ML. 2012). The oligonucleotides may have four domains, though in some examples, an oligonucleotide can have 1, 2, 3, 4, 5, 6, 7, 8, 9, 10, 11, 12, 13, 14, 15, 16, 17, 18, 19, 20, or more domains. The term " domains" relates to a strand associate into a DNA arm junction.

In one example, some oligonucleotides are 4-domain oligonucleotides, and each domain hybridizes to a domain in separate oligonucleotides. The nucleic acid nanostructure of the invention may be a branched nucleic acid structure that comprises four individual DNA strands portions of which are complementary in a specific pattern (i.e., "Holliday junctions") (Lilley DM 2000). A duplex is formed by hybridization of complementary domains, each domain located on one neighbouring oligonucleotide (one domain on one oligonucleotide binds to another domain in another nearby oligonucleotide). Typically, an approximately 90° dihedral angle is formed by hybridization of complementary domains, each domain located on physically separate oligonucleotides. In one example, the nanostructure is comprised of nucleic acid duplexes (i.e., a pair of hybridized or partially hybridized oligonucleotides), with adjacent duplexes connected by a single phosphate bond (i.e., "crossover"). In one example, a plurality of single-stranded oligonucleotides anneals to form adjacent duplexes. In some instance during self-assembly, a 4-domain oligonucleotide adopts a helical conformation such that two (of the four) domains reside in one duplex and the remaining two domains reside in an adjacent duplex. In one example, two antiparallel DNA duplexes lie next to each other, and share two junction points where strands cross from one duplex into the other (i.e. "double crossover" or "DX motif") (Seemann NC 2004). The antiparallel orientation of the nucleic acid helical domains in antiparallel DX motifs implies that the major grooves of one nucleic acid helix faces the minor groove of the other engaged helices come together in each turn.

The term "strand", as used herein, relates to a nucleic acid strand, e.g. a DNA strand, an RNA strand, a modified DNA strand, a modified RNA strand, a RNA strand, a LNA strand and/or a combination thereof. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein.

The principle for designing, for example, self-assembled nucleic acid nanostructures is that sequence complementarity in nucleic acid strands is selected such that, by pairing up complementary segments, the nucleic acid strands self-organize into a predefined nanostructure under appropriate physical conditions. Nanostructures may be formed using any nucleic acid hybridization methodology (Rothemund PWK 2006; Douglas SM et al. 2009; Han D et al. 2011). One such methodology is DNA origami. In a DNA origami approach, a nanostructure is produced by the folding of a longer "scaffold" nucleic acid strand through its hybridization to a plurality of shorter "staple" oligonucleotides, each of which hybridize to two or more non-contiguous regions within the scaffold strand. The scaffolding strand may be linear or circular. The scaffolding strand may be naturally or non-naturally occurring. The nanostructure, e.g. DNA origami nanostructure which is composed of one or multiple origami subunits ("entities"), may comprise at least one scaffolding strand, i.e. single-stranded polynucleotide scaffold DNA with a known sequence.

The DNA origami structure may further comprise a plurality of single-stranded oligonucleotide staple strands, wherein each staple strand may be at least partially complementary to at least one scaffolding strand. The term "staple strand", as used in this invention, shall refer to a single-stranded oligonucleotide molecule, which is at least partially complementary to at least one scaffolding strand, wherein the at least one scaffolding strand may upon hybridization with a plurality of staple strands be folded and/or arranged such that the desired nanostructure may be formed. Further, each of the staple strands may be configured to bind to the at least one scaffolding strand, wherein the at least one scaffolding strand may be folded and/or arranged such that the desired nanostructure may be formed.

DNA origami are nucleic acid nanostructures that can be designed to have a predetermined structure. For example, nucleic acid nanostructures may be designed prior to their synthesis, and their size, shape, complexity and modification may be prescribed and controlled using certain selected oligonucleotides in the synthesis process. The location of each nucleic acid in the structure may be known and provided for before synthesizing a nanostructure of a particular shape. The fundamental principle for designing, for example, self-assembled nucleic acid nanostructures is that sequence complementarity in nucleic acid strands is selected such that, by pairing up complementary segments, the nucleic acid strands self-organize into a predefined nanostructure under appropriate physical conditions. Such designs may for example be performed using software such as caDNAno. CaDNAno estimates DNA as slices, with each slice representing a base pair (Castro et al. 2011). However, it should be understood that the present technology is not limited thereto, and it should be understood that this is merely exemplary and that also other nanostructures may be utilized to exercise the present invention.

The term "oligonucleotide" is intended to include, but not limited to, a polymeric form of nucleotides that may have various lengths and comprise deoxyribonucleotides or ribonucleotides or analogues thereof. An oligonucleotide is typically composed of a specific sequence of four nucleotide bases: adenine (A); cytosine (C); guanine (G); and thymine (T) (uracil (U) for thymine (T) when the polynucleotide is RNA). Thus, the term "oligonucleotide sequence" is the alphabetical representation of a polynucleotide molecule; alternatively, the term may be applied to the polynucleotide molecule itself. Oligonucleotides may optionally include one or more non-standard nucleotide(s), nucleotide analog(s) and/or modified nucleotides.

The term "plurality" as used herein in the context of oligonucleotides, is defined to mean any number of two or more, for example in the range between two and 3000, preferably 50-1000, 100-500, or 200-400, more preferably 20-40. A plurality of oligonucleotides may include one copy of each oligonucleotide. However, it is also possible that several copies of the same oligonucleotide are present. An exemplary set of oligonucleotides comprises two or more copies of each oligonucleotide. The term "comprises a plurality of single-stranded oligonucleotides", as used herein, refers to assembled from a plurality of single-stranded oligonucleotides.

According to the invention, at least two of the plurality of single-stranded oligonucleotides of the at least one nucleic acid nanostructure each comprise a first identical portion, i.e. at least two oligonucleotides of the nucleic acid nanostructure have the same nucleobase sequence. In one example, the nucleobase sequence can be identical over the whole length. In one example, the first identical nucleobase sequence can be a subsequence of a given sequence, i.e. a sequence that can be derived from a given sequence by deleting some elements without changing the order of the remaining elements.

As used herein, the term "comprising" is to be construed as encompassing both "including" and "consisting of', both meanings being specifically intended, and hence individually disclosed embodiments in accordance with the present invention.

The term "at least two", as used herein in the context of plurality of oligonucleotides, shall include at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or any other number.

In one example, said first identical nucleobase sequence is part of the staple strands. For example, the at least two single-stranded oligonucleotides of the nucleic acid nanostructure having each a first identical nucleobase sequence may be identified using a sequencing method, for example, PCR.

In accordance with this invention, DNA single strand molecules of defined sequence and length, in particular single stranded DNA oligonucleotides and polypeptides, can be prepared from several linear or circular sources of polynucleotides, such as dsDNA, ssDNA, and synthesized or naturally occurring oligonucleotides as, for example, described in WO 2018/054571 A1, WO 2019/094928 A1, US 20230272461 A1, WO 2023/041931 A1, WO 2015/079042 A1, and Monsur Ali et al.

The term "at least one molecule capable of specifically binding to at least one analyte" refers to at least one binding molecule or reporting moiety configured to bind to at least one analyte or a target, e.g. at least one analyte in a sample or a target on a cell surface. The term " at least one molecule capable of specifically binding to at least one analyte" or "binding molecule" or " reporting moiety " are used interchangeably and relate to at least one molecule capable of specific binding at least one analyte or a target. In accordance with this invention, the interaction between at least one molecule capable of specifically binding to at least one analyte and its target/analyte is characterized by the specific binding. The term "specifically binding" or "specific", as used herein, means that at least one molecule capable of specifically binding to at least one analyte or binding molecule, e.g. antibody, is capable of specifically interacting with and/or binding to at least one specific analyte/target or a set of specific analytes/targets but does not essentially bind to other molecules, such as antigens. Such binding may be exemplified by the specificity of a lock-and-key-principle. When referring to the term "binding", such term is to be construed to comprise the term "specific binding". Preferably, the interaction between at least one molecule capable of specifically binding to at least one analyte and its analyte or target is characterized by the specific binding of an antibody-antigen interaction and/or a ligand-receptor interaction. The person skilled in the art is able to identify similar specific interactions, which shall also be within the scope of this invention. As an example, the specific binding of an aptamer to a molecule, or of a receptor ligand to its receptor, may also be used, or *vice versa.*

The term "analyte" or "target", as used herein, relates to any analyte or a target molecule, such as a chemical compound, a peptide, a protein, an antigen, lipid, and/or any other molecule to be bound by at least one molecule capable of specifically binding to at least one analyte, e.g. a surface peptide, surface protein, surface receptor, surface antigen, and/or surface lipid of a target. In one example, the analyte is a molecule presented on the surface of a target and/or accessible on the surface of the target. In one example, an analyte such as an antigen is a cell-surface bound molecule or a sensor-surface bound molecule, such as any of proteins, cluster-of-differentiation molecules, receptors, lipids, ion channels, sugars, or any other cell-surface bound molecule or structure. In one example, an analyte is an antigen. For example, an analyte can be a sensor, an immobilization surface, a bead, a column material of a chromatography column, microtiter plate surface, a biotinylated or streptavidin-coated surface, and/or any other surface, material, or molecule used for an assay to immobilize analytes such as cells.

Conjugates of the invention are advantageous because such conjugates can be used in many established *in vitro* and *in vivo* assays, and in existing high-throughput platforms from industry leaders achieving high specificity and sensitivity. The conjugate of the invention allows the detection and/or quantification of analytes in a sample with a high dynamic range of a signal and minimized non-specific interaction to address novel targets or low abundance analytes or biomarkers due to enhanced reagent performance with high specificity and sensitivity. For example, such conjugates of the invention comprising nanostructures, advantageously allow for quick and efficient adjustment to different indications, biochemical and diagnostic assays, and settings. Due to the use of at least two single-stranded oligonucleotides each comprising a first identical nucleobase sequence, the conjugate of the invention is high sensitivity and suitable for detecting and/ or quantifying analytes, novel targets or low abundance analytes or biomarkers in the femtomolar range, e.g. using qPCR. Furthermore, the conjugates of the invention are advantageous because they comprise biodegradable DNA nanostructures that are non-xenobiotic and have numerous merits such as good stability, strong predictability, excellent programmability, and easy synthesis. The use of the invention, the method of the invention, and the kit of the invention benefit from the above-mentioned advantages of conjugates of the invention comprising a nanostructure.

In one example, said at least one molecule capable of specifically binding to at least one analyte of (a) and/or said at least one nanostructure of (b) do(es) not carry a radiation emitting label or a detectable label. The term "radiation emitting label", as used herein, relates to any molecule or dye molecule that can be used to generate signals selected from fluorescence, luminescence, radiation, enzyme and particle, in particular a visible or a non-visible particle. In one example, said radiation emitting label is any molecule or dye molecule selected from electromagnetic radiation and radioactive radiation. In a preferred example said electromagnetic radiation is fluorescent or luminescent. In a further preferred example said radioactive radiation is gamma radiation. The term "detectable label", as used herein, relates to a molecule and/or a nanoparticle such as a functionalized nanoparticle, which has a functional substituent attached to it. In one example, the detectable label is selected from the group comprising an enzyme label, fluorescent label, and a particle label, in particular a visible or a non-visible particle label, e.g. a fluorescent particle, a liposome, a gold particle, a latex particle, a Q-Dot, a carbon nanotube, a silver particle, a silver coated particle, a cellulose particle. The detectable labels can be chosen from the group of gold nanoparticles, silver nanoparticles, metal nanoparticles, dyed polystyrene or latex beads, enzyme or fluorophores, i.e., fluorescent labels, e.g., fluorescent particles, a Q-Dot, a carbon nanotube, a silver coated particle, a cellulose particle. The radiation emitting label or the detectable label can be detected by using any microscopy or imaging technique known to a person skilled in the art. In one example, the radiation emitting label or the detectable label cannot be used in a method of the third aspect of the invention. In an example, said first identical nucleobase sequence is the only feature intended for subsequent detection and/or quantification.

When referring to a nanostructure, such nanostructure relates to the nanostructure itself such as a DNA origami but does not relate to the at least one molecule capable of specifically binding to at least one analyte.

In one embodiment, said at least one molecule capable of specifically binding to at least one analyte of (a) is selected from an antibody or derivative thereof, a small molecule, a fynomer, an aptamer, a peptide nucleic acid (PNA), a polypeptide, a peptide, a glycoprotein, a peptidomimetic, an anticalin, an Affilin, an Affimer, an Affitin, an Alphabody, a nanobody, a DARPin, a receptor ligand, a receptor, a T cell receptor (TCR)-like antibody, a MHC molecule, a pMHC molecule, a receptor domain, a functional fragment or derivative thereof, and combinations thereof.

In one example, the at least one molecule capable of specifically binding to at least one analyte is any of an antibody or an antigen-binding peptide thereof, for example an IgG, a Fab fragment, a single chain Fab fragment, or a single domain antibody, a DNA and/or RNA aptamer, a peptide, a binding protein, a ligand of a cell-surface receptor (e.g. PD-IA), or a lipid, e.g. any of an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a DNA aptamer, a RNA aptamer, a peptide nucleic acid (PNA), a polypeptide, a peptide, a glycoprotein, a peptidomimetic, an anticalin, an Affilin, an Affimer, an Affitin, an Alphabody, a nanobody, DARPin, a receptor ligand, a receptor, a T cell receptor (TCR)-like antibody, a major histocompatibility complex (MHC), a peptide-loaded major histocompatibility complex (pMHC), a receptor domain, and a functional fragment or derivative thereof, and combinations thereof.

In one example, the least one molecule capable of specifically binding to at least one analyte is a bi- or multispecific molecule such as bi- or multispecific antibodies. In one example, a molecule capable of specifically binding to an analyte is an antibody or antigen-binding fragment thereof, an antigen-binding peptide, a Fab fragment, a F(ab')2 fragment, a Fv fragment, a diabody, a single chain Fv fragment, a (scFv)2, a tetrabody, a triabody, a disulfide bond-stabilized Fv (dsFv), a Fc domain, an engineered Fc domain, a TCR-like antibody, a MHC, a pMHC, a receptor ligand, a functional fragment or derivative thereof, and combinations thereof.

The term "antibody" has its art-established meaning and refers to any immunoglobulin, including antibodies and fragments thereof, that binds to a specific antigen. The term includes polyclonal, monoclonal, chimeric, single domain (Dab) and bi- or multispecific antibodies, or derivatives thereof. As used herein, antibody or antibody molecule contemplates recombinantly generated intact immunoglobulin molecules and immunologically active portions of an immunoglobulin molecule such as, without limitation: Fab, Fab', F(ab')2, F(v), scFv, scFv2, scFv-Fc, minibody, diabody, tetrabody, single variable domain (e.g., variable heavy domain, variable light domain), bispecific, and peptabodies. "F(v)" is an antibody fragment which contains an antigen-recognition and -binding site. Antibody fragments are known in the art and include Fab fragments and single chain variable fragments (scFv). This region consists of a dimer of one heavy- and one light-chain variable domain in tight, non-covalent association. It is in this configuration that the three CDRs of each variable domain interact to define an antigen-binding site on the surface of the VH-VL dimer. Collectively, the six CDRs confer antigen-binding specificity to the antibody.

Antibody fragments and derivatives are reviewed in, for example, Nelson, 2010. Antibody derivatives may have more than one binding specificity. Nanobodies are derived from the antigen-binding variable heavy chain regions of heavy chain antibodies found in camels and llamas, which lack light chains.

Antibodies and fragments thereof are accordingly capable of binding to a given antigen. Preferably such binding is specific, i.e., the affinity of an antibody or fragment thereof for a given antigen (the cognate antigen) is higher than for any other possible antigen. For example, a molecule capable of specifically binding to an analyte can be an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic. A preferred antibody is immunoglobulin G (IgG).

The term "small molecule", as used herein, refers to any molecule, organic compound or chemical entity, with a molecular weight of less than about 5,000 Daltons (Da), preferably within a mass range of about 50 to about 1500 Daltons, typically involved in a biological process as a substrate or product. For example, such small biological molecules can be fatty acids, glucose, amino acids, and cholesterol and secondary metabolites such as lipids, glycosides, alkaloids, and natural phenols.

In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

The term "fynomer", as used herein, relates to small binding proteins derived from the SH3 domain of Fyn kinase with a molecular weight of 7 kDa that can be engineered to bind to yield specific and high-affinity binding domains to target specific proteins. Fynomers are described in, for example, Grabulovski et al., 2007.

As used herein, the term "aptamer" refers to a peptide and/or nucleic acid that substantially specifically binds to a specified substance. In the case of a nucleic acid aptamer, the aptamer is characterized by binding interaction with a target other than Watson/Crick base pairing or triple helix binding with a second and/or third nucleic acid. Such binding interaction may include Van der Waals interaction, hydrophobic interaction, hydrogen bonding and/or electrostatic interactions, for example. Similarly, peptide-based aptamers are characterized by specific binding to a target wherein the aptamer is not a naturally occurring ligand for the target. As an example, the specific binding of an aptamer to a molecule, or of a receptor ligand to its receptor, may also be used, or *vice versa.*

Affilins are described in, for example, Ebersbach et al., 2007. Affimers are described in, for example, Johnson et al., 2012. Affitins are described in, for example, Krehenbrink, 2008. Alphabodies are described in, for example, Desmet et al., 2014. DARPins are described in, for example, Stumpp et al., 2008. MHC molecules are described in, for example, Janeway et al., 2001.

For example, the at least one molecule capable of specifically binding to at least one analyte can be an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic. For example, a MHC is a MHC class I or a MHC class II. In one embodiment, a pMHC is a pMHC class I or a pMHC class II.

In one embodiment, said at least one molecule capable of specifically binding to at least one analyte of (a) is covalently or non-covalently, such as electrostatically, bound to said nucleic acid nanostructure, and/or wherein at least two of said nanostructures of (b) are covalently or non-covalently, such as electrostatically, bound to each other.

As used herein, the term "bound" refers to both covalent interactions and non-covalent interactions. A covalent interaction is a chemical linkage between two atoms or radicals formed by the sharing of a pair of electrons (i.e., a single bond), two pairs of electrons (i.e., a double bond) or three pairs of electrons (i.e., a triple bond). Covalent interactions are also known in the art as electron pair interactions or electron pair bonds. Non-covalent interactions include, but are not limited to van der Waals interactions, hydrogen bonds, weak chemical bonds (i.e., via short - range non-covalent forces), hydrophobic interactions, ionic bonds, electrostatic interactions and the like. For example, a molecule capable of specifically binding to an analyte reversibly or irreversibly binds to its analyte/target. For example, reversibly molecules capable of specifically binding to an analyte may be an antibody, an antibody fragment, a DNA strand, a biotin molecule, a streptavidin molecule, whereas irreversibly binding molecules may be a maleimide-thiol chemistry molecule or a click chemistry molecule.

In one embodiment, at least one binding site of said molecule of (a) capable of specifically binding to at least one analyte, preferably for all binding sites in the case said molecule of (a) with more than one binding site, is at a distance from a surface of said nanostructure of about 3 nm to about 30 nm, preferably at a distance from a surface of said nanostructure of about 7 nm to about 9 nm.

The term "at a distance of 3 nm to 30 nm from a surface of said nanostructure", as used herein, means that said at least one molecule capable of specifically binding to at least one analyte is attached to said nanostructure, preferably via a linker, wherein a distance between the said molecule and a surface of the nanostructure, e.g. a distance provided by the length of a linker, is in the range of about 3 nm to about 30 nm. For example, said molecule of (a) is located about 3 nm to about 15 nm away from said nanostructure, but is bound to said nanostructure, e.g. via a linker. The advantage of such a distance is that the at least one molecule capable of specifically binding to at least one analyte is highly accessible, and improved accessibility over at least one analyte/at least one targeting agent which does not have such distance is provided.

The term "accessible", as used herein, means capable of binding to and/or being bound by at least one molecule capable of specifically binding to at least one analyte. The term at least one molecule capable of specifically binding to at least one analyte being "accessible", in the context of a surface, means that such surface does not sterically hinder the accessibility of such at least one molecule capable of specifically binding to at least one analyte. Such a spacing, i.e. distance, between at least one molecule capable of specifically binding to at least one analyte and a surface of a nanostructure has the advantage of reducing steric hindrance.

The term "surface", as used herein, relate to surfaces of a nanostructure. In one example, a "surface" relates to a surface which comprises, for example, the form of an area, line, edge, vertex, and/or DNA helix blunt end.

In one embodiment, said nanostructure of (b) has at least one vertex, and the point of attachment of said molecule of (a) to said nanostructure of (b) is about 2 nm or less away from the vertex of said nanostructure of (b). Accordingly, a point of attachment, e.g. a position where said molecule of (a) is directly attached and/or a position where a linker, e.g. a molecule capable of specifically binding to an analyte-bound linker (i.e. a linker bound to at least one molecule capable of specifically binding to at least one analyte) is attached, has a distance of < 5 nm, preferably < 2 nm from a vertex, or edge, of said nanostructure of (b). The advantage of such a distance is that said molecule of (a) is highly accessible, and improved accessibility over at least one analyte/at least one targeting agent which does not have such proximity to a vertex or edge is provided.

In one example, at least one molecule capable of specifically binding to at least one analyte is attached to the nanostructure of (b), for example via a linker. In one example, said at least one molecule capable of specifically binding to at least one analyte is not part the nanostructure of (b) but is attached to the core structure e.g. a DNA origami, optionally via a staple strand conjugated to the binding molecule. For example, when the nanostructure is at least partially formed by a DNA origami structure and wherein the DNA origami structure comprises at least one scaffolding strand and a plurality of staple strands, the at least one molecule capable of specifically binding to at least one analyte may be bound to one of the at least one scaffolding strand or a staple strand by means of a linker molecule, wherein the linker molecule may be connected to a DNA strand portion, which is complementary to a portion of the at least one scaffolding strand or to a portion of a staple strand.

In one example, a linker molecule is one or more nucleic acid bases, e.g. a single nucleic acid base. For example, a linker molecule of a molecule capable of specifically binding to an analyte is connected to a DNA strand of said nanostructure. For example, a binding site comprises a linker and/or a site configured to bind to a linker. For example, a linker has at least 16, preferably at least 20, more preferably at least 26 nucleic acid bases.

In one embodiment, said nanostructure of (b) is a three-dimensional nanostructure. In a preferred embodiment, the nanostructure of (b) is an origami nanostructure, such as a DNA origami structure, as exemplarily disclosed in US 7,842,793 B2.

The term "DNA origami structure", as used herein, relates to a nanostructure that comprises DNA as a building material to make nanoscale shapes. Preparing and/or providing a DNA origami involves folding of one or more "scaffolding" DNA strands into a particular shape using a plurality of "staple" DNA strands, e.g. by self-assembly. The nucleic acid sequences of the staple strands are designed such that the staple strands hybridize to particular portions of the scaffolding strands and, due to the hybridization, a particular shape of the nanostructure is obtained as disclosed, for example, in Zadegan RM and Norton ML 2012.

In one embodiment, said nanostructure of (b) comprises at least one single-stranded scaffolding strand, preferably the strand is a separate nucleic acid molecule, with said plurality of single-stranded oligonucleotides being hybridized thereto. In one example, a scaffolding strand makes up and/or traverses the main part of a DNA origami structure and/or a DNA origami subunit ("entity"). In one example, an entity is a DNA origami subunit. In one example, each of the staple strands is configured to bind to at least one of the at least one scaffolding strand in at least one, preferably two or more distinct places.

In one embodiment, said at least one single-stranded scaffolding strand has a length of more than about 100 bases, and said plurality of single-stranded oligonucleotide staple strands have a length of from about 20 to about 100 bases.

In one example, said at least one single-stranded scaffolding strand has a length of from about 100 to about 20000 nucleic acid bases, preferably from about 120 to about 10000 nucleic acid bases. In one example, the length of said plurality of single-stranded oligonucleotide staple strands can be between about 40 nucleotides to about 100 nucleotides in length, preferably about 50 nucleotides. For example, a nucleic acid nanostructure and/or a DNA origami comprised by a nucleic acid nanostructure comprise(s) ≤ 100 staple strands. For example, a nucleic acid nanostructure and/or a DNA origami structure comprise(s) ≥ 10 DNA strands, preferably ≥ 15 DNA strands, e.g. at least one scaffolding strand and at least nine staple strands.

In one embodiment, said nanostructure of (b) has a maximum extension smaller than 1000 nm, preferably smaller than 500 nm, such as around 100 nm, or smaller. In one example, the nanostructure comprises a maximum extension in the range of from about 10 nm to about 70 nm, preferably about 20 nm to about 50 nm. The nanostructure of this invention can be of any extension.

The term "maximum extension", as used herein, relates to a maximum length of the nanostructure, preferably along the longitudinal axis. In one example, a maximum extension of the nanostructure along a transverse axis is smaller than the maximum extension of the nanostructure which is the maximum extension along a longitudinal axis. In one example, the maximum extension is smaller than the maximum longitudinal extension. The term "longitudinal axis", as used herein, relates to a line passing through a maximum extension and/or a maximum length of a nanostructure, e.g. a maximum extension, and/or a principle axis of inertia. The term "transverse axis", as used herein, relates to an axis that is transverse to a longitudinal axis, e.g. perpendicular to a longitudinal axis and/or is a crosswise axis to a longitudinal axis.

For example, the shape of a nanostructure may be any shape, for example, a brick, a rod, a triangular shape, a round shape, a cuboid, i.e. a rectangular shape, a star shape, or any other shape.

In addition, the DNA origami structures can be stabilized against low salt or nuclease degradation. In one embodiment, the nucleic acid nanostructure of b) comprises a protective coating, said coating preferably comprising or consisting of an oligolysine-polyethylene glycol (PEG) copolymer, preferably K10-PEG oligolysine. A nanostructure may be stabilized against nuclease degradation, against disassembly by low ionic-strength environments, and/or *in vivo* conditions using coatings as described in "Oligolysine-based coating protects DNA nanostructures from low-salt denaturation and nuclease degradation", (Ponnuswamy et al. 2017 and WO 2015/070080), and/or stabilization methods described in "Sequence-programmable covalent bonding of designed DNA assemblies" (Gerlin T. 2018 and WO 2019/234122). Advantageously, the conjugate of the invention comprising DNA origami structure can be stabilized against nucleases. A further advantage of the conjugate of the invention comprising a nanostructure is that it is stable in any buffer and even in samples such as blood samples.

In one embodiment, at least two, preferably all, of said first identical nucleobase sequences are hybridized to a first complementary strands. The term "first complementary strands", as used herein, refer to a single-stranded oligonucleotide molecule, which is complementary to the identical nucleobase sequence. In one example, the first complementary strands is complementary to the first identical nucleobase sequence. In one example, the first complementary strands is a PCR-tag used for amplification in PCR reactions. In a further example, the first identical nucleobase sequences is a PCR-tag used for amplification in PCR reactions. In one example, said first complementary strands or said first identical nucleobase sequences are the only features intended for subsequent detection and/or quantification. In a further example, the first complementary strands refer to a primer binding site, i.e. at least one primer attaches to the first complementary strands. In an example, the first complementary strands are identical to each other. In one example, the first complementary stands have a length of from about 20 to about 40 bases, preferably a length of about 20 bases. In a further example, the first complementary strands have a length over a sufficient number of bases to bind at least two first identical nucleobase sequence adjacent to each other in at least one nucleic acid nanostructure of b). For example, the complementary strands may have a total length of about 20 to about 40 bases, and the at least two first identical nucleobase sequence may have each a total length of about 10 to about 20 bases, respectively, wherein the complementary strands can bind to both first identical nucleobase sequences. For example, the complementary strands may have a total length of about 40 bases, and the at least two first identical nucleobase sequence may have each a total length of about 20 bases, respectively, wherein the complementary strands can bind to both first identical nucleobase sequences. For example, the complementary strands may have a total length of about 20 bases, and the at least two first identical nucleobase sequence may have each a total length of about 10 bases, respectively, wherein the complementary strands can bind to both first identical nucleobase sequences.

For example, the complementary strands may have a total length over a sufficient number of bases to bind at least two unique primers. In one example, the first complementary strands is partially hybridized to the first identical nucleobase sequence. In one example, the complementary sequence is longer than the first identical sequence and thus, only partly complementary to the first identical sequence. In one example, the first complementary sequence has a length of about 30 to about 100 bases, preferably about 40 to 60 about bases, more preferably about 40 to about 50 bases. In a further example, the first identical sequence has a length of about 10 to 40 bases, preferably about 20 to about 30 bases.

The term "primers" as used herein relates to short single stranded oligonucleotides which are complementary to the 3' sequences of the positive and negative strand of the target sequence. Usually, distinct sets of primers are employed for each sequence being amplified. The reaction mix is cycled in repeated heating and cooling steps. The heating cycle denatures or splits a double stranded nucleic acid target into single stranded templates. In the cooling cycle, the primers bind to complementary sequence on the template. After the template is primed the nucleic acid polymerase creates a copy of the original template. Primers are those that are capable of binding to a known binding region of the target polynucleotide (here the first complementary strands) and facilitating ligation of an oligonucleotide probe. Sequencing primers may be designed with the aid of a computer program such as, for example, DNAWorks, Gene2Oligo, or Nucleic Acid Package (NUPACK). The binding region can vary in length, but it should be long enough to hybridize the sequencing primer. In one example, the binding region has a length of about 40 bases. In one example, the primer has a length of about 20 bases.

In one embodiment, in said nanostructure of b), at least two of said oligonucleotides each comprise a second identical nucleobase sequence, wherein preferably said first and said second identical nucleobase sequences are present in the same copy number in said nanostructure of (b), wherein more preferably each occurrence of said first and said second identical nucleobase sequence are adjacent to each other in said nanostructure of (b). In a preferred example, at least two of said oligonucleotides comprise a second identical nucleobase sequence but do not comprise a first identical nucleobase sequence. In one example, said first complementary strands or said second complementary strands are the only features intended for subsequent detection and/or quantification.

In one embodiment, in at least two of said oligonucleotides, preferably in all occurrences of said first and said second identical nucleobase sequences, both first and second identical nucleobase sequences are hybridized to one second complementary strand, i.e. the second complementary strand hybridized with both first and second identical nucleobase. In one example, the second complementary strands is partially hybridized to the first identical nucleobase sequences and second identical nucleobase sequences.

In one example, the second complementary sequence has a length of about 30 to about 100 bases, preferably about 40 to 60 about bases, more preferably about 40 to about 50 bases. In an example, the first identical sequence has a length of about 10 to 40 bases, preferably about 20 to about 30 bases. In a further example, the second identical sequence has a length of about 10 to 40 bases, preferably about 20 to about 30 bases.

In one example, said second complementary strands, as used herein, refer to a PCR-tag used for amplification in PCR reactions. In one example, said first and said second complementary strands are PCR-tags used for amplification in PCR reactions, respectively. In one example, said second identical nucleobase sequence, as used herein, refer to a PCR-tag used for amplification in PCR reactions.

In one example, said one second complementary strands have a length over a sufficient number of bases to bind both first and second identical nucleobase sequences adjacent to each other. For example, said one second complementary strands may have a total length of about 20 to about 40 bases, and the first and second identical nucleobase sequences may have each a total length of about 10 to about 20 bases, respectively, wherein the one second complementary strands can bind to both first and second identical nucleobase sequences. For example, the one second complementary strands may have a total length of about 40 bases, and the first and second identical nucleobase sequences may have each a total length of about 20 bases, respectively, wherein the said one second complementary strands can bind to both first and second identical nucleobase sequences. For example, the one second complementary strands may have a total length of about 20 bases, and the first and second identical nucleobase sequences may have each a total length of about 10 bases, respectively, wherein the one second complementary strands can bind to both first and second identical nucleobase sequences.

In one example, the first and the second identical nucleobase sequence do not hybridize to the nanostructure and extend off from the nanostructure of b) to form overhangs, called "sticky ends". In one example, said first and said second complementary strands do not hybridize to the nanostructure and extend off from the nanostructure of b) to form overhangs, called "sticky ends".

### Use of a conjugate for detection and quantification an analyte

In a second aspect, the present invention provides a use of a conjugate of the first aspect for detecting and/or quantifying at least one analyte.

Preferred embodiments of the conjugate of the first aspect define, where applicable, preferred embodiments of the use of the second aspect.

In one example, when referring to a use, the use is an *in vivo, ex vivo, in vitro,* or *in situ* use, e.g. an *in vitro* use. In one example, the nanostructure of the invention is for use *in vivo* or *in vitro,* preferably *in vivo.*

### Method of detecting and/or quantifying an analyte

In a third aspect, the present invention provides a method of detecting and/or quantifying at least one analyte, said method comprising:
(a) allowing formation of a complex of a conjugate as defined above in the first aspect and said at least one analyte; and
(b) in said complex, detecting and/or quantifying the amount of said first complementary strands or said second complementary strands, respectively.

In one example, the present invention provides a method of detecting and/or quantifying at least one analyte, said method comprising:
(a) allowing formation of a complex of a conjugate as defined above in the first aspect and said at least one analyte; and
(b) in said complex, detecting and/or quantifying the amount of said first identical nucleobase sequence.

Step (a) of the method of detecting and/or quantifying at least one analyte, e.g. in a sample, provides targeting at least one analyte or target with a conjugate of the first aspect of the invention comprising (a) at least one molecule capable of specifically binding to at least one analyte, and (b) at least one nanostructure, thereby allowing the formation of a complex of a conjugate and an analyte.

The term "complex" as used herein, relates to a complex of said conjugate of the first aspect of the invention and at least one analyte.

The term "analyte" or "target" as used herein relates to any molecule, structure, material, cell, antigen and/or surface to be targeted. In one example, said analyte is a cell, molecule, material, and/or surface e.g. a sensor surface, immobilization surface, or any other surface used for an assay, e.g. an immuno-assay, preferably an immuno-sandwich assay or a bead-based immuno-assay. An "immuno-assay" as used herein is a biochemical test that measures the presence or concentration of at least one analyte or target in a solution through, in particular the use of the conjugate of the fist aspect of the invention. For example, an analyte can be a sensor, an immobilization surface, a bead, a column material of a chromatography column, a microtiter plate surface, a biotinylated or streptavidin-coated surface, and/or any other surface, material, or molecule used for an assay, e.g. an immuno-assay, preferably an immuno-sandwich assay, to immobilize analytes.

In one example, after the step (a) allowing the formation of a complex of the conjugate of the first aspect of the invention with the at least one nanostructure of b) and at least one analyte, and prior to the step (b) of detecting and/or quantifying the amount of said oligonucleotides each comprising a first complementary strands or second complementary strands, unbound conjugate of the first aspect of the invention can be removed, e.g., by performing a washing step or using precipitation such as PEG-precipitation, filtration such as ultra-filtration, and/or liquid chromatography such as High-performance liquid chromatography (HPLC) as described in detail in the examples and in Stahl et al. 2014.

Due to its high sensitivity, the method of the present invention is suitable for detecting and/or quantification of novel targets, low abundance analytes or biomarkers analytes in the femtomolar range.

In one embodiment, said detecting and/or quantifying comprises, to the extent only first complementary sequences are present, amplifying said first complementary sequences by polymerase chain reaction (PCR). In one example, said detecting and/or quantifying comprises amplifying said first identical nucleobase sequence by polymerase chain reaction (PCR). In a preferred embodiment, said PCR is quantitative PCR (qPCR) or multiplexed PCR. In a more preferred embodiment, amplifying is affected with primers specific for said first complementary sequence, respectively.

In one embodiment, said detecting and/or quantifying comprises, to the extent first and second complementary sequences are present, amplifying said one second complementary strands which comprises said first and second complementary sequences by polymerase chain reaction (PCR). In a preferred embodiment, said PCR is quantitative PCR (qPCR) or multiplexed PCR. In a more preferred embodiment, amplifying is affected with primers specific for said first or second complementary sequence, respectively.

In one example, said first complementary strands or second complementary strands are the only features intended for subsequent detection and/or quantification. In one example, said first identical nucleobase sequence is the only features intended for subsequent detection and/or quantification.

The term "Polymerase Chain Reaction" or "PCR" refers usually to a method of exponential amplification of specific nucleic acid target in a reaction mix with a nucleic acid polymerase and primers. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known in the art. For example, in a conventional PCR using Taq DNA polymerase, a double stranded target nucleic acid may be denatured at a temperature greater than 90 °C, primers annealed at a temperature in the range 50-75 °C, and primers extended at a temperature in the range 68-78 °C. The term "PCR" encompasses derivative forms of the reaction, including but not limited to, RT-PCR, real-time PCR, nested PCR, quantitative PCR, multiplexed PCR, assembly PCR and the like. Reaction volumes range from a few hundred nanoliters, e.g. 200 nL, to a few hundred microliters, e.g. 200 µL.

According to the invention, step (b) of the method of detecting and/or quantifying at least one analyte provides detecting and/or quantifying the amount of said first complementary strands, said second complementary strands, or said first identical nucleobase sequence in a sample, e.g. a cell sample, a tissue sample, blood sample, and/or body fluid sample. In one example, detecting and/or quantifying in step (b) comprises analyzing the presence of a complex of a conjugate as defined in the first aspect and at least one analyte in a blood sample, body-fluid sample, tissue sample, and/or organ sample obtained from said subject, e.g. by identifying of the first complementary strands or said second complementary strands or said first identical nucleobase sequence of the at least one nucleic acid nanostructure of b).

In one example, said detecting and/or quantifying in step (b) comprises sequencing said first complementary strands, said second complementary strands, or said first identical nucleobase sequence. In one example, the step (b) of detecting and/or quantifying at least one analyte comprises determining a signal obtained from decoding said first complementary strands, said second complementary strands, or said first identical nucleobase sequence. For example, decoding said first complementary strands, said second complementary strands, or said first identical nucleobase sequence may comprise sequencing, e.g. using qPCR or multiplex PCR.

In one embodiment, said PCR is quantitative PCR (qPCR) or multiplexed PCR. The term "quantitative PCR" or "qPCR", as used herein, means a PCR designed to measure the abundance of one or more specific target sequences in a sample or specimen. Techniques for quantitative PCR are well-known in the art. As used herein, the term "qPCR" relates to a process of monitoring a PCR reaction by recording the fluorescence generated either by an intercalating dye such as SYBR^{®} Green or a target-specific reporter probe at each cycle. This is generally performed on a Real-Time PCR instrument that executes thermal cycling of the sample to complete the PCR cycles and data specified point in each cycle measures the fluorescence of the sample in each channel through a series of excitation/emission filter sets. The term "multiplex PCR" as used herein refers to a method in which multiple specific primers are used allowing detection of several analytes by a single assay. In one example, a response is qualitatively or quantitatively determined using sequencing or PCR, preferably quantitative PCR (qPCR).

According to the invention, conjugates of the first aspect comprising at least one nucleic acid nanostructure of b) can be contacted with a sample, and the oligonucleotides each comprising a first and/or second identical nucleobase sequence can be identified by determining said first complementary strands, said second complementary strands, or said first identical nucleobase sequence i.e. showing a desired response in said sample. Since the at least two oligonucleotides of the at least one nucleic acid nanostructure of b) of the first aspect each comprise the same first and/or second identical nucleobase sequence hybridized to said first complementary strands and/or said second complementary strands, the first and/or second identical nucleobase sequence can serve as a unique identifier. For example, detecting and/or quantifying the amount of said first complementary strands, said second complementary strands, or said first identical nucleobase sequence are used to correlate a determined response. In one example, said first complementary strands, said second complementary strands, or said first identical nucleobase sequence anywhere within the nanostructure may be identified for a given cycle of hybridization and/or detection and/or quantification. In an example, said first complementary strands, said second complementary strands, or said first identical nucleobase sequence are the only features intended for subsequent detection and/or quantification.

In one example, the method of detecting and/or quantifying at least one analyte using a conjugate of first aspect of the invention comprising (a) at least one molecule capable of specifically binding to at least one analyte and (b) at least one nucleic acid nanostructure comprises the formation of a complex in a desired assay, e.g. immune-assay, preferably immuno-sandwich assay. In one example, said first complementary strands, said second complementary strands, or said first identical nucleobase sequence are the only features intended for subsequent detection and/or quantification in a desired assay, preferably immuno-sandwich assay or a bead-based immuno-assay.

In one example, the method of detecting and/or quantifying at least one analyte does not comprise a radiation emitting label or a detectable label as defined in the first aspect. The term "radiation emitting label", as used herein, relates to any molecule or dye molecule that can be used to generate signals selected from fluorescence, luminescence, radiation, enzyme and particle, in particular a visible or a non-visible particle. In one example, said radiation emitting label is any molecule or dye molecule selected from electromagnetic radiation and radioactive radiation. In a preferred example said electromagnetic radiation is fluorescent or luminescent. In a further preferred example said radioactive radiation is gamma radiation. The term "detectable label", as used herein, relates to a molecule and/or a nanoparticle such as a functionalized nanoparticle, which has a functional substituent attached to it. In one example, the detectable label is selected from the group comprising an enzyme label, fluorescent label, and a particle label, in particular a visible or a non-visible particle label, e.g. a fluorescent particle, a liposome, a gold particle, a latex particle, a Q-Dot, a carbon nanotube, a silver particle, a silver coated particle, a cellulose particle. The detectable labels can be chosen from the group of gold nanoparticles, silver nanoparticles, metal nanoparticles, dyed polystyrene or latex beads, enzyme or fluorophores, i.e., fluorescent labels, e.g., fluorescent particles, a Q-Dot, a carbon nanotube, a silver coated particle, a cellulose particle.

In a further example, the method of detecting and/or quantifying at least one analyte does not comprise a radiation emitting label or a detectable label in an immune-assay, wherein the immune-assay is selected from the group consisting of an enzyme-linked immunosorbent assay (ELISA), a radioimmunoassay (RIA), electrochemiluminescence assay (ECL), a fluorescence polarization immunoassay (FPIA), an enzyme multiplied immunoassay technique (EMIT), luminescent oxygen channeling immunoassay (LOCI), kinetic interaction of microparticle in solution (KIMS), and particle enhanced turbidimetric inhibition immunoassay (PETINIA). In a further example, the method of detecting and/or quantifying at least one analyte does not comprise an immune-assay selected from the group consisting of an enzyme-linked immunosorbent assay (ELISA), a radioimmunoassay (RIA), electrochemiluminescence assay (ECL), a fluorescence polarization immunoassay (FPIA), an enzyme multiplied immunoassay technique (EMIT), luminescent oxygen channeling immunoassay (LOCI), kinetic interaction of microparticle in solution (KIMS), and particle enhanced turbidimetric inhibition immunoassay (PETINIA).

In one example, the method of detecting and/or quantifying at least one analyte using a conjugate of first aspect of the invention comprises performing such method in a well plate known in the art, for example in a 384-well plate or in a 94-well plate, preferably in a 384-well plate, wherein at least one analyte or at least one target is tested per well.

In one example, the at least one analyte to be detected and/or to be quantified using the method of the invention is preferably selected from analytes present in a clinical sample, a biological, or environmental sample. In particular, examples of nucleic acid analytes are genomic DNA, mRNA or products derived therefrom, e.g. cDNA. The sample may be any sample which may contain the at least one analyte to be detected. For example, the sample may be a clinical sample, such as a tissue sample or a body fluid sample such as blood, serum, plasma etc., particularly of human origin. Further types of samples include but are not limited to a biological sample such as an agricultural sample, environmental samples, soil samples, food samples, forensic samples or samples from valuable goods which are tested for brand protection.

In one example, the method of detecting and/or quantifying at least one analyte comprises determining and/or quantitatively measuring said first complementary strands or said second complementary strands occurrence in organs or blood samples obtained, preferably minimally or non-invasively obtained, by sequencing or PCR, preferably qPCR.

In one embodiment, amplifying is affected with primers specific for said first or second complementary sequence, respectively.

In one example, at least two, preferably all of said first identical nucleobase sequence are hybridized to first complementary strands or said second complementary strands. A probe with a signaling moiety, such as SYBR^{®} Green, may then hybridized to the first complementary strands or said second complementary strands and the signaling moiety can be detected or quantified using PCR, in particular qPCR. This occurs only during the qPCR reaction.

In an example, the PCR method involves the use of fluorescently labeled oligonucleotides (short DNA molecules). The probe is usually labeled both at the 5' end and at the 3' end. A fluorescent reporter is placed at the 5' end of the probe and a fluorescent quencher at the 3' end of the probe. As long as the reporter is in proximity to the quencher, no fluorescence will be detected. Thus, fluorescence is usually detected only after the 5' end and the 3' end of the fluorescently labeled oligonucleotide probe separate. This usually occurs during the qPCR reaction as a byproduct of the enzymatic activity of the polymerase when the probe incorporates into the PCR product.

In one example, when referring to a method, the method is an *in vivo, ex vivo, in vitro,* or *in situ* method, e.g. an *in vitro* method.

### A kit for the detection and/or quantifying an analyte

In a fourth aspect, the present invention provides a kit for the detection and/or quantifying at least one analyte comprising or consisting of a conjugate as defined in the first aspect and primers as defined in the second aspect specific for said first or said second complementary strands.

It will be appreciated, that the use of the singular article "a" or "the" within this document is not meant to limit the scope of the invention expect if specifically stated. That is, generally "a" may also refer to more than one. In other words, "a" can generally be read as "at least one".

The term "at least one", as used herein, shall include at least 1, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or any other number.

The terms "of the (present) invention", "in accordance with the invention", "according to the invention" and the like, as used herein, are intended to refer to all aspects and embodiments of the invention described and/or claimed herein.

### BRIEF DESCRIPTION OF THE FIGURES

The present invention is now further described by reference to the following figures.

All methods mentioned in the figure descriptions below were carried out as described in detail in the examples.

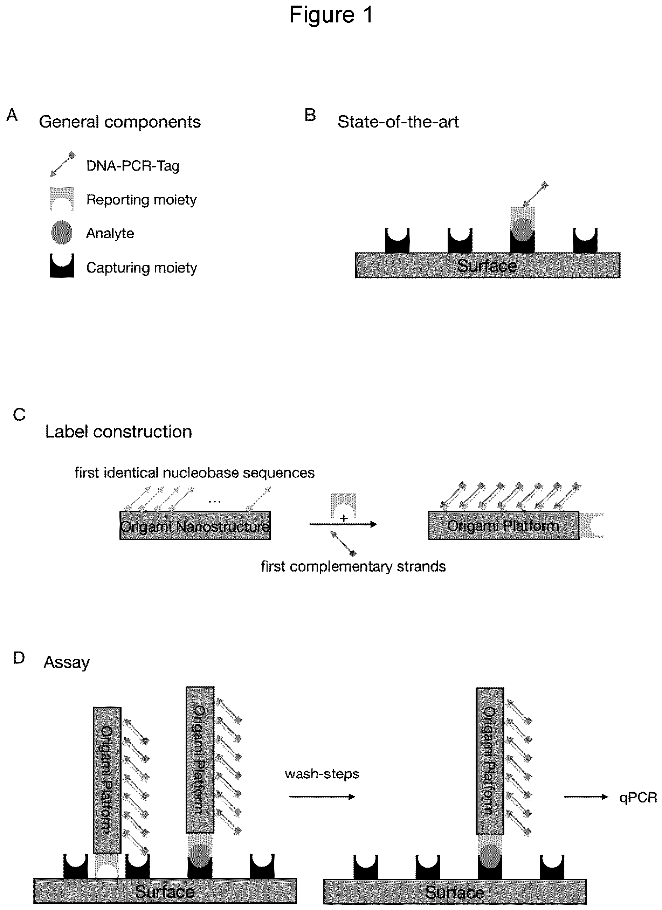

**Figure 1** describes the construction of a conjugate to the first aspect of the invention for PCR according. (A) describes the components needed for an immuno-sandwich, capturing moiety, molecule of interest (at least one analyte), reporting moiety (at least one molecule capable of specifically binding to at least one analyte or binding molecule) which generates the signal, and DNA-PCR-tags. **(B)** illustrates the formation of immuno-sandwich with a reporting moiety presenting one PCR-tag. **(C)** depicts the construction of reporting moiety based on the DNA-origami technology. Here the platform has multiple staple strand overhangs (first identical nucleobase sequences) which are complementary to PCR-tag, and the origami platform can bind to the capturing moiety. **(D)** Shows the PCR assay and the formation of an immuno-sandwich, which was captured but also unbound particles (left). After the wash steps, only successful sandwiches are left, and a PCR can be performed (right).

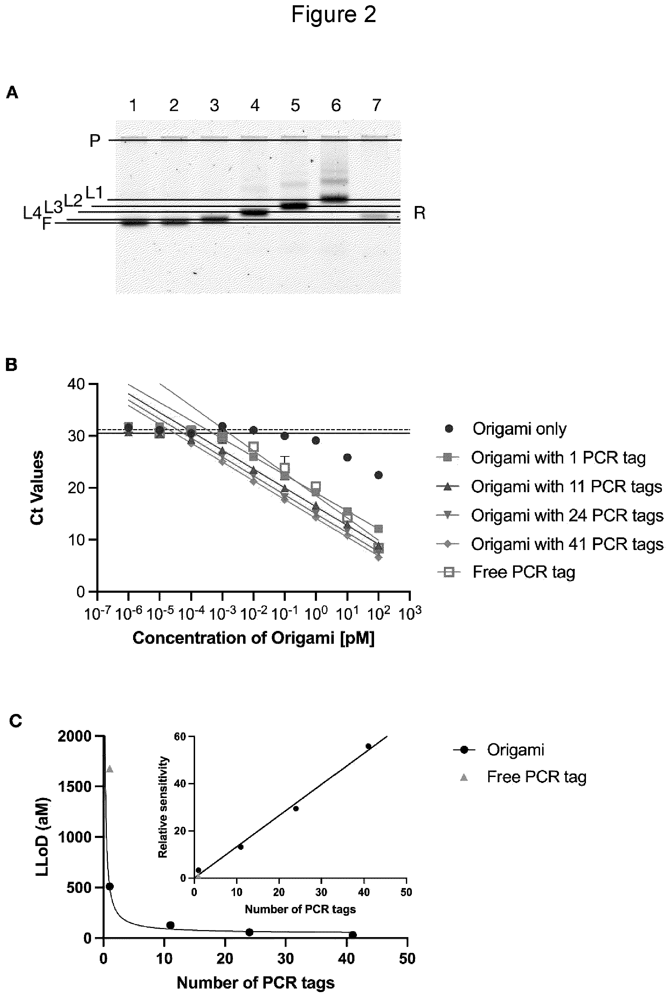

**Figure 2** shows the qPCR-results for different conjugates to the first aspect of the invention (DNA-origami labels). (A) is a fluorescent image of an agarose gel on which different versions of conjugates to the first aspect of the invention (the origami-label) were electrophoretically separated: 1, origami only; 2, origami-all-off (i.e., only PCR-tags in solution); 3, two label; 4, 12 label; 5, 25 label; 6, 42 label; 7, ref. P, gel pocket; L1-to L4 mark bands of the different labels, F marks the band of the fully folded platform without label and R marks the band of a reference. (B) shows Ct-values as a function of the origami or free-PCR-tag concentration in solution. Different conjugates to the first aspect of the invention (origami-platforms) with different numbers of PCR-tags and free PCR-tag were analyzed by qPCR as indicated in the legend above. The dashed black line indicates blank measurement (Ct = 31.2), the solid black line indicates the blank minus three standard deviations of the blank (Ct = 30.5), other lines are fits of the relation Ct = B+A*log(c) to the respective data, where A and B are fit parameters and c is the concentration. **(C)** shows the lower limit of detection (LLoD) versus the number of PCR tags on the origami as derived from the concentrations where the fits intersect with the blank minus three standard deviations. The inset depicts the relative sensitivity which was calculated as the inverse LLoD and then normalized to the free PCR tag sensitivity. Thus, the origami variant with 41 PCR tags has a 56 times improved sensitivity compared to the free PCR tag. The solid line is a linear fit to the data points of the origami variants, supporting a linear relation between the number of PCR tags and the increase in sensitivity.

### EXAMPLES

In the following, reference is made to the examples, which are given to illustrate, not to limit the present invention.

### Example 1: Folding of DNA-origami Nanostructures

The reaction mixtures contained scaffold DNA at a concentration of e.g. 50 nM (can vary with requirements) and oligonucleotide strands at 4-fold molar excess each according to Sandhöfer N et al. 2015. The reaction buffer included 5 mM TRIS, 1 mM EDTA, 5 mM NaCl (pH 8) and 10 to 20 mM MgCh. The reaction mixtures were subjected to a thermal annealing ramp using TETRAD (MJ Research, now Biorad) thermal cycling devices. Oligonucleotides (staple strands and staple strands with overhangs), and PCR-tags were obtained from IDT. DNA scaffolds were produced in-house according to Engelhardt et al. 2019. See table below for folding ramps used to assemble the objects. After assembly the objects were purified from excess stands according to Stahl et al. 2014. After the purification steps the PCR-tag was added in molar excess at 37°C for 30 min. Afterwards the platform was further modified with binders e.g. antibodies as described in publication Wagenbauer et al 2023. Finally, the label with tags and binders was purified from excess DNA and binders via e.g. HPLC (see Example 2).

| **Object** | **Scaffold type (length in bases)** | **Highest temperature for 30 min (°C)** | **Ramp** | **Incubation temperature (°C)** |
|---|---|---|---|---|
| DNA Nanostructure 1 | 8064 | 65 | 55 to 50; 1°C/1h | 25 |
| DNA Nanostructure 2 | 1033 | 65 | 56 to 54 1°C/2h | 25 |

### Example 2: Purification, enrichment, and in vitro stabilization of DNA Nanostructures

After the folding reaction, all reaction products were purified using PEG-precipitation, size exclusion HPLC or ultra-filtration depending on the purpose as describes in Stahl et al. 2014. All procedures were performed as described in Wagenbauer et al. 2017. Concentrations of DNA-origami objects were analyzed with a Nanodrop 8000 (Thermo Fisher). Before using the objects in different assays, the objects were stabilized for the use in low-ionic strength buffers and the presence of nucleases. To that end, we used the protocol by Ponnuswamy et al. 2017 and coated all our structures with K10-PEG oligolysine, purchased from Alamanda Polymers (USA).

### Example 3: Gel electrophoresis

Folded DNA-nanostructures (unpurified or purified) were electrophoresed on 1.5% to 3.5% agarose gels containing 0.5× TBE and MgCl₂ at different concentrations for around 2 h at 70 V bias voltage in a gel box immersed in a water bath if not otherwise specified. The electrophoresed agarose gels were scanned using a Typhoon FLA 9500 laser scanner (GE Healthcare) at 50 µm/px resolution. The resulting 16-bit tif-images were analyzed using a customized Matlab-based analysis software and/or ImageJ 1.440.

### Example 4: DNA-Antibody conjugation, antibody digestion, and attachment to DNA-Origami objects

Attachment of antibody-DNA conjugates to DNA-origami objects: Antibody-DNA conjugates and DNA-origami objects with the corresponding binding sites were incubated in equimolar ratios for 1h at 37°C as described in Wagenbauer et al. 2023.

### Example 5: PCR Assay

Assay performance:
1. Dilution of samples towards the upper detection limit of the qPCR device (100 pM)
2. Preparation of the MasterMix (18 µL) was as follows:

| Ingredient | volume |
|---|---|
| PowerUp SYBR Green | 10 µL |
| ddH₂O | 6 µL |
| Primer (Forward and Reverse) | 2 µL |

3. The final reaction volume in qPCR tube (20 µL) was as follows:

| Ingredient | volume |
|---|---|
| MasterMix | 18 µL |
| Sample | 2 µL |

4. Seal the well plate and spin down
5. Start qPCR device and chose the right program
6. Analysis

### RESULTS

### Construction of a DNA-origami label for PCR:

The analyte of interest was brought into contact with the conjugate of the first aspect of the invention comprising (a) at least one molecule capable of specifically binding to at least one analyte (reporting moiety) and (b) at least one nucleic acid nanostructure (here, an origami nanostructure) having multiple staple strand overhangs which are complementary to PCR-tags (Figure 1 C). An immuno-sandwich was formed, wherein the at least one analyte of interest was sandwiched between a) the capture moiety coated onto the wells of a plate, beads, or any other platform and b) the conjugate of the first aspect of the invention. The PCR-tags were analyzed by qPCR (Figure 1 D).

### PCR - Results for different DNA-origami labels:

Fluorescent image of an agarose gel (Figure 2A): Different versions of the origami-labels (nucleic acid nanostructure of the fist aspect of the invention) were electrophoretically separated according to their molecular weights: Origami only (1), origami-all-off (2), and the origami with two labels (3) migrated the fastest, followed by the origami with 12 labels (4), the origami with 25 labels (5), and the origami with 42 labels (6). Therefore, the origami with two labels runs faster than the origami with 42 labels.

Ct-values as a function of the origami (nucleic acid nanostructure of the fist aspect of the invention) concentration in solution (Figure 2B): decreasing Ct-values were measured with increasing concentration of origamis with different numbers of PCR-Tag in solution. Origami with one PCR-Tag had a higher CT-value compared to Origami with 41 PCR-Tags in solution.

The dilution series were fitted using a logarithmic relation between the Ct value Ct and the concentration of the origami or free tag c (Figure 2C): Ct = B+A*log(c), where A and B are fitting parameters. The lower limit of detection (LLoD) for each variant was calculated from the intersection of the fit with the Ct value of the blank (31.2) minus three standard deviations (0.24). The LLoD an inversely related to the number of PCR tags on the origami. In addition, the sensitivity as 1/LLoD was calculated and normalized to the free-PCR-tag control. The sensitivity is proportional to the number of PCR tags. Therefore, the sensitivity of can be increased by increasing the number of PCR tags on the origami platform.

### REFERENCES

Castro et al. A primer to scaffolded DNA origami. Nature Methods 8.3, p221-229, 2011.
Douglas, S. M. et al. Self-assembly of DNA into nanoscale three-dimensional shapes. Nature 459, 414-418, 2009.
Ebersbach et al., Affilin-Novel Binding Molecules Based on Human γ-B-Crystallin, an All β-Sheet Protein. J. Mol. Biol. 372, 172-185, 2007.
Engelhardt FA, Praetorius F, Wachauf CH, Briiggenthies G, Kohler F, Kick B, et al., Custom-size, functional, and durable DNA origami with design-specific scaffolds. ACS nano 2019, 13(5): 5015-5027.
Gerling T. Sequence-programmable covalent bonding of designed DNA assemblies. Science Advances, Vol. 4, 1157, 2018.
Grabulovski et al., A Novel, Non-immunogenic Fyn SH3-derived Binding Protein with Tumor Vascular Targeting Properties. The Journal of Biological Chemistry 282, 3196-3204, 2007.
Han, D. et al. DNA origami with complex curvatures in three-dimensional space. Science 332, 342-346, 2011.
Janeway et al., Immunobiology: The Immune System in Health and Disease (5th ed.). New York: Garland Science, 2001.
Johnson et al., Sensitive Affimer and Antibody Based Impedimetric Label-Free Assays for C-Reactive Protein Anal. Chem. 84, 6553-6560, 2012.
Koirala D. Shrestha P. Emura T. Hidaka K. Mandal S. Endo M. Sugiyama H. Mao H. Single-molecule mechanochemical sensing using DNA origami nanostructures. Angew. Chem. Int. Ed., 8137-8141,2014.
Krehenbrink et al., Artificial Binding Proteins (Affitins) as Probes for Conformational Changes in Secretin PulD. J Mol Biol 383, 1058-1068, 2008.
Lilley DM. Structures of helical junctions in nucleic acids. Q Rev Biophys.,33(2):109-59, 2000.
Monsur Ali M. et al. Rolling circle amplification: a versatile tool for chemical biology, materials science and medicine. Chem. Soc. Rev. 43, 3324-3341, 2014.
Nelson A.L., Antibody fragments: hope and hype, Mabs. 2, 77-83, 2010.
Ponnuswamy N. Oligolysine-based coating protects DNA nanostructures from low-salt denaturation and nuclease degradation. Nat Commun., 8:15654, 2017.
Rinker S. Ke Y. Liu Y. Chhabra R. Yan H. Self-assembled DNA nanostructures for distance-dependent multivalent ligand-protein binding. Nat. Nanotechnol., 418-422, 2008.
Rothemund, P. W. K. Folding DNA to create nanoscale shapes and patterns. Nature 440, 297-302, 2006. Sandhöfer N, Metzeler K, Rothenberg M, Herold T, Tiedt S, Groiss V, et al. Dual PI3K/mTOR inhibition shows antileukemic activity in MLL-rearranged acute myeloid leukemia. Leukemia 2015, 29(4): 828-838.
Seeman NC. Nanotechnology and the double helix. Sci Am., 290(6):64-9, 72-5, 2004.
Stahl E, Martin TG, Praetorius F, Dietz H.
Facile and scalable preparation of pure and dense DNA origami solutions. Angewandte Chemie 2014, 53(47): 12735-12740.
Stumpp et al., DARPins: a new generation of protein therapeutics. Drug Discov Today 13, 695-701, 2008.
Wagenbauer KF, Engelhardt FAS, Stahl EK, Hechtl VK, Stommer P, Seebacher F, et al. How we make DNA origami. Chembiochem: a European journal of chemical biology 2017.
Wagenbauer KF et al. Programmable multispecific DNA-origami-based T-cell engagers. Nat Nanotechnol., 18(11):1319-1326, 2023
Wang D. Vietz C. Schröder T. Acuna G. Lalkens B. Tinnefeld P. A DNA walker as a fluorescence signal amplifier. Nano Lett., 5368-5374, 2017.
Wang S. Zhou Z. Ma N. Yang S. Li K. Teng C. Ke Y. Tian Y. DNA origami-enabled biosensors. Sensors, 20, 6899, 2020.
Zadegan RM, Norton ML. Structural DNA nanotechnology: from design to applications. Int J Mol Sci.;13(6):7149-7162. 2012.
Zhang Z. Wang Y. Fan C. Li C. Li Y. Qian L. Fu Y. Shi Y. Hu J. He L. Asymmetric DNA origami for spatially addressable and index-free solution-phase DNA chips. Adv. Mater., 2672-2675, 2010.
Zhang Z. Zeng D. Ma H. Feng G. Hu J. He L. Li C. Fan C. A DNA-Origami chip platform for label-free SNP genotyping using toehold-mediated strand displacement. Small, 1854-1858, 2010.

## Claims

1. A conjugate comprising or consisting of
(a) at least one molecule capable of specifically binding to at least one analyte; and
(b) at least one nucleic acid nanostructure;
wherein said nucleic acid nanostructure of (b) comprises a plurality of single-stranded oligonucleotides; and wherein at least two of said plurality of oligonucleotides each comprise a first identical nucleobase sequence.

2. The conjugate of any one of the preceding claims, wherein said molecule of (a) is selected from an antibody or derivative thereof, a small molecule, a fynomer, an aptamer, a peptide nucleic acid (PNA), a polypeptide, a peptide, a glycoprotein, a peptidomimetic, an anticalin, an Affilin, an Affimer, an Affitin, an Alphabody, a nanobody, a DARPin, a receptor ligand, a receptor, a T cell receptor (TCR)-like antibody, a MHC molecule, a pMHC molecule, a receptor domain, a functional fragment or derivative thereof, and combinations thereof.

3. The conjugate of any one of the preceding claims, wherein said molecule of (a) is covalently or non-covalently, such as electrostatically, bound to said at least one nanostructure (b), and/or wherein at least two of said nanostructures of (b) are covalently or non-covalently, such as electrostatically, bound to each other.

4. The conjugate of any one of the preceding claims, wherein a binding site of said molecule of (a) capable of specifically binding to an analyte is at a distance from a surface of said nanostructure of about 3 nm to about 30 nm, preferably at a distance from a surface of said nanostructure of about 7 nm to about 9 nm, optionally wherein said nanostructure of (b) has at least one vertex, and wherein the point of attachment of said molecule of (a) to said nanostructure of (b) is about 2 nm or less away from a vertex of said nanostructure.

5. The conjugate of any one of the preceding claims, wherein said nanostructure of (b) is a three-dimensional nanostructure, preferably an origami nanostructure such as a DNA-origami nanostructure.

6. The conjugate of any one of the preceding claims, wherein said nanostructure of (b) comprises at least one single-stranded scaffolding strand with said plurality of single-stranded oligonucleotides being hybridized thereto, optionally wherein said at least one single-stranded scaffolding strand has a length of more than about 100 bases, and wherein said plurality of single-stranded oligonucleotide staple strands have a length of from about 20 to about 100 bases.

7. The conjugate of any one of the preceding claims, wherein said nanostructure of (b) has a maximum extension smaller than 1000 nm, preferably smaller than 500 nm.

8. The conjugate of any one of the preceding claims, wherein said nanostructure of (b) comprises a protective coating, said coating preferably comprising or consisting of an oligolysine-polyethylene glycol (PEG) copolymer, preferably K10-PEG oligolysine.

9. The conjugate of any one of the preceding claims, wherein at least two, preferably all of said first identical nucleobase sequences, are hybridized to the first complementary strands.

10. The conjugate of any one of the preceding claims, wherein in said nanostructure of (b), at least two of said oligonucleotides each comprise a second identical nucleobase sequence, wherein preferably said first and said second identical nucleobase sequences are present in the same copy number in said nanostructure of (b), wherein more preferably each occurrence of said first and said second identical nucleobase sequence are adjacent to each other in said nanostructure of (b), optionally wherein in at least two of said oligonucleotides, preferably in all occurrences of said first and said second identical nucleobase sequences, both first and second identical nucleobase sequences are hybridized to one second complementary strand.

11. Use of the conjugate of any one of claims 1 to 10 for detecting and/or quantifying at least one analyte.

12. A method of detecting and/or quantifying at least one analyte, said method comprising
(a) allowing formation of a complex of a conjugate of claims 1 to 10 and said at least one analyte; and
(b) in said complex, detecting and/or quantifying the amount of said first complementary strands or said second complementary strands, respectively.

13. The method of claim 12, wherein said detecting and/or quantifying comprises, to the extent only first complementary sequences are present, amplifying said first complementary sequences by polymerase chain reaction (PCR), preferably wherein said PCR is quantitative PCR (qPCR) or multiplexed PCR, more preferably wherein amplifying is affected with primers specific for said first or second complementary sequence, respectively.

14. The method of claim 12, wherein said detecting and/or quantifying comprises, to the extent first and second complementary sequences are present, amplifying said one second complementary strands which comprises said first and second complementary sequences by polymerase chain reaction (PCR), preferably wherein said PCR is quantitative PCR (qPCR) or multiplexed PCR, more preferably wherein amplifying is affected with primers specific for said first or second complementary sequence, respectively.

15. A kit for the detection and/or quantifying at least one analyte, said kit comprising or consisting of the conjugate of any one of claims 1 to 10 and primers as defined in claim 13 or 14.
